# EUROPEAN PATENT SPECIFICATION

(11) **EP 4 355 042 B1**
(45) Date of publication and mention of the grant of the patent: **25.03.2026**
(21) Application number: 22815035.5
(22) Date of filing: 18.05.2022
(51) Int. Cl.: A61B 5/02, A61B 5/0205, A61B 5/00, G04G 17/00, G04G 21/02, G06F 1/16, A61B 5/024, A61B 5/1455, B29C 70/88

(54) **METHOD FOR MAKING COVER PLATE, COVER PLATE AND ELECTRONIC DEVICE**
HERSTELLUNGSVERFAHREN FÜR ABDECKPLATTE, ABDECKPLATTE UND ELEKTRONISCHE VORRICHTUNG
PROCÉDÉ DE FABRICATION DE PLAQUE DE RECOUVREMENT, PLAQUE DE RECOUVREMENT ET DISPOSITIF ÉLECTRONIQUE

(30) Priority: 01.06.2021 CN 202110610012
(43) Date of publication of application: 17.04.2024
(73) Proprietor: GUANGDONG OPPO MOBILE TELECOMMUNICATIONS CORP., LTD., Dongguan, Guangdong 523860 (CN)
(72) Inventor: XU, Feng, Dongguan, Guangdong 523860 (CN); YANG, Zimei, Dongguan, Guangdong 523860 (CN); TANG, Zhongzhi, Dongguan, Guangdong 523860 (CN); LIN, Jianwen, Dongguan, Guangdong 523860 (CN); WU, Yingchao, Dongguan, Guangdong 523860 (CN)
(74) Representative: Santarelli
(86) International application number: PCT/CN2022/093619
(87) International publication number: WO 2022/252988

(56) References cited:
- CN-A- 109 917 557
- CN-A- 109 917 557
- CN-A- 111 973 167
- CN-A- 113 301 752
- CN-A- 113 301 753
- CN-U- 212 118 134
- CN-U- 212 118 134
- TW-A- 201 436 393
- US-A1- 2020 305 794
- US-A1- 2021 076 545

## Description

### TECHNICAL FIELD

The present disclosure relates to the field of electronic technologies, and in particular to a method for making a cover plate, a cover plate, and an electronic device.

### BACKGROUND

Optical components inside electronic devices may be configured to detect environmental parameters or human body parameters. For example, the health functions of a smart watch include a blood oxygen detection function. The current mainstream blood oxygen detection method is photoplethysmography (PPG). The operating principle of PPG is as follows. Light rays emitted by a light emitter enter the outside world through a transparent area of a housing of a smart watch, and then pass through tissues and arterial veins in a skin. A part of the light rays is absorbed and another part of the light rays is reflected back to a light sensor. Light signals received by the light sensor are converted into electrical signals, and blood oxygen data is obtained after conversion.

US2021/076545A1 discloses a method according to the preamble of claim 1 and a cover plate according to the preamble of claim 8. It provides an electronic device that includes a housing defining an aperture and at least partially defining an internal volume of the electronic device, an electromagnetic radiation emitter and an electromagnetic radiation detector disposed in the internal volume, and an optical component disposed in the aperture. The optical component includes a first and second transparent portions disposed above the electromagnetic radiation detector and the electromagnetic radiation emitter, and an opaque portion disposed between the first and second transparent portions. A conductive component is in electrical communication with the opaque portion and one or more components of the electronic device.

An existing structure of the PPG generally includes a housing, and the housing includes a back cover. The back cover is provided with a first optical window and a second optical window. The optical signals emitted by the light emitter may pass through the first optical window to reach the skin, and the light signals returning from the skin may pass through the second optical window to enter the light sensor. However, the light rays emitted by the light emitter and emitted into the first optical window may pass through the second optical window and then received by the light sensor. That is, the light signals emitted by the light emitter are directly received by the light sensor without being absorbed and reflected by the skin, which leads to deviations in the calculation results, thereby reducing the accuracy of the detection results.

### SUMMARY OF THE DISCLOSURE

The embodiments of the present disclosure provide a method for making a cover plate, a cover plate, and an electronic device. The invention is set out in the appended set of claims.

### BRIEF DESCRIPTION OF THE DRAWINGS

In order to more clearly illustrate the technical solutions in some embodiments of the present disclosure, hereinafter, a brief introduction will be given to the accompanying drawings that are used in the description of some embodiments.
FIG. 1 is a flow chart of a method for making a first type of cover plate in some embodiments of the present disclosure.
FIG. 2 is a schematic process flow chart of a first type of method for making a composite rod material in the method shown in FIG. 1.
FIG. 3 is a schematic process flow chart of a second type of method for making the composite rod material in the method shown in FIG. 1.
FIG. 4 is a schematic process flow chart of processing the composite rod material prepared by the method as shown in FIG. 1 to obtain a cover plate.
FIG. 5 is a flow chart of a method for making a second type of cover plate in some embodiments of the present disclosure.
FIG. 6 is a schematic process flow chart of a first type of method for making the composite rod material in the method shown in FIG. 5.
FIG. 7 is a schematic process flow chart of a second type of method for making the composite rod material in the method shown in FIG. 5.
FIG. 8A is a schematic process flow chart of a first type of method for making a first composite rod body in the method shown in FIG. 5.
FIG. 8B is a schematic process flow chart of a second type of method for making a first composite rod body in the method shown in FIG. 5.
FIG. 9A is a schematic process flow chart of a first type of method for making a second composite rod body in the method shown in FIG. 5.
FIG. 9B is a schematic process flow chart of a second type of method for making a second composite rod body in the method shown in FIG. 5.
FIG. 10 is a schematic process flow chart of a third type of method for making the composite rod material in the method shown in FIG. 5.
FIG. 11 is a schematic process flow chart of processing the composite rod material prepared by the method as shown in FIG. 5 to obtain the cover plate.
FIG. 12 is a structural schematic view of the cover plate in some embodiments of the present disclosure.
FIG. 13 is a partial structural schematic view of an electronic device in some embodiments of the present disclosure.
FIG. 14 is a partial structural schematic view of the electronic device shown in FIG. 13.

### DETAILED DESCRIPTION

Referring to the drawings, the same reference numerals represent the same components. The principle of the present disclosure is illustrated by being implemented in a suitable environment. The following description is based on some embodiments illustrated in the present disclosure, which should not be considered as limiting other embodiments of the present disclosure that are not described in detail here.

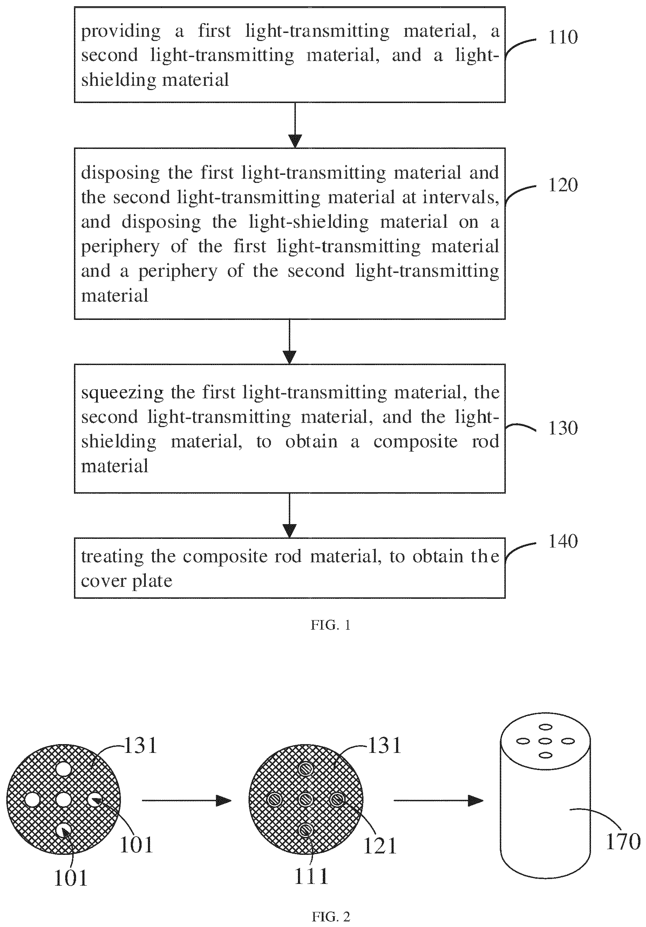

As illustrated in FIG. 1, FIG. 1 is a flow chart of a method for making a first type of cover plate in some embodiments of the present disclosure. The method for making the first type of cover plate includes the following operations.

At block 110, a first light-transmitting material, a second light-transmitting material, and a light-shielding material are provided.

Each of the first light-transmitting material and the second light-transmitting material may be selected from at least one of transparent glass, transparent plastic, a transparent ceramic, and a sapphire. The transparent plastic may be polymethyl methacrylate (PMMA). In some embodiments, a light transmittance of the first light-transmitting material to light rays of 400 nm-1200 nm is greater than or equal to 20%, such as 20%, 30%, 40%, 50%, 60%, 70%, 80%, 90%, or 100%. A light transmittance of the second light-transmitting material to the light rays of 400 nm-1200 nm is greater than or equal to 20%, such as 20%, 30%, 40%, 50%, 60%, 70%, 80%, 90%, or 100%.

The first light-transmitting material and the second light-transmitting material may be the same material or different materials.

The light-shielding material may be selected from at least one of opaque glass, opaque plastic, an opaque ceramic, and a metal. The opaque plastic may be polycarbonate (PC), polyamide (PA), polyetheretherketone (PEEK), etc., and a color of the "opaque" may be black or dark. In some embodiments, a light transmittance of the light-shielding material to the light rays of 400 nm-1200 nm is less than or equal to 10%, such as 10%, 9%, 8%, 7%, 6%, 5%, 4%, 3%, 2%, 1%, or 0.

At block 120, the first light-transmitting material and the second light-transmitting material are disposed at intervals, and the light-shielding material is disposed on a periphery of the first light-transmitting material and a periphery of the second light-transmitting material.

As illustrated in FIG. 2, FIG. 2 is a schematic process flow chart of a first type of method for making a composite rod material in the method shown in FIG. 1. A light-shielding material 131 may be a single opaque rod material, and the light-shielding material 131 defines a plurality of hollow structures 101 that are disposed at intervals. In this case, disposing the light-shielding material on the periphery of the first light-transmitting material and the periphery of the second light-transmitting material, may include: disposing a first light-transmitting material 111 and a second light-transmitting material 121 in different hollow structures 101, respectively. Each of the hollow structures 101 may be a through hole or a groove. The number of the hollow structures 101 in the light-shielding material 131 is at least 2, and may be more than 2, such as 3, 4, 5, 6, 7, 8, etc.

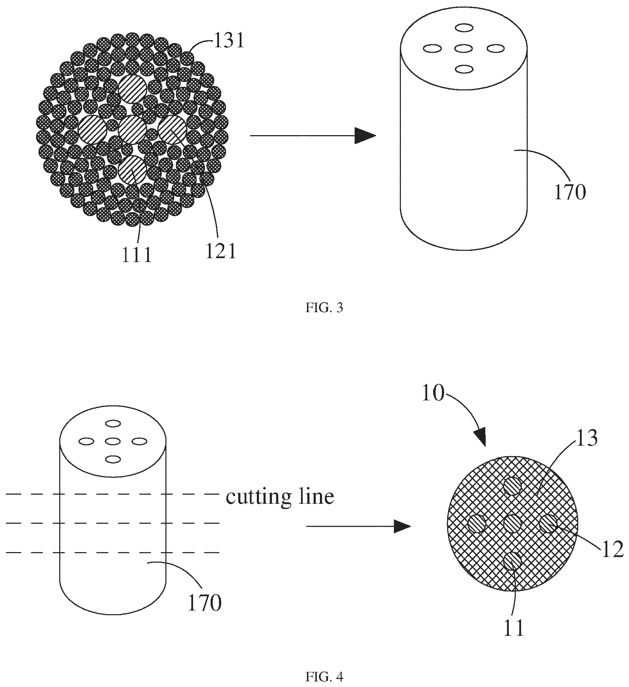

As illustrated in FIG. 3, FIG. 3 is a schematic process flow chart of a second type of method for making the composite rod material in the method shown in FIG. 1. The light-shielding material 131 may include a plurality of opaque fiber filaments. In this case, the disposing the light-shielding material on the periphery of the first light-transmitting material and the periphery of the second light-transmitting material, may include: arranging the plurality of opaque fiber filaments on the periphery of the first light-transmitting material 111 and the periphery of the second light-transmitting material 121. When the light-shielding material 131 adopts the opaque rod material with an integral structure, it is necessary to punch holes on the light-shielding material 131 to form the hollow structures 101. For materials that are difficult to process, such as glass, there are often problems such as difficulty in drilling and cracking of the rod material, thereby increasing processing difficulty. The light-shielding material 131 adopts the plurality of opaque fiber filaments, which avoids drilling, thereby reducing the processing difficulty, improving the production yield, and reducing the production cost. In the present disclosure, the "fiber filament" is only configured to indicate that a length of a material is greater than a cross-sectional width of the material, which does not limit the specific fineness of the material.

The first light-transmitting material 111 may be a single first light-transmitting rod material, or the first light-transmitting material 111 includes a plurality of first light-transmitting fiber filaments. The second light-transmitting material 121 may be a single second light-transmitting rod material, or the second light-transmitting material 121 includes a plurality of second light-transmitting fiber filaments.

At block 130, the first light-transmitting material, the second light-transmitting material, and the light-shielding material are squeezed, to obtain the composite rod material.

As illustrated in FIG. 2 and FIG. 3, the light-shielding material 131 may be deformed under the action of an external force and at a room temperature. For example, when the light-shielding material 131 is the metal or the plastic that has plasticity at the room temperature, the first light-transmitting material 111, the second light-transmitting material 121, and the light-shielding material 131 may be combined by squeezing without heating. Of course, the first light-transmitting material 111, the second light-transmitting material 121, and the light-shielding material 131 may also be heated to improve the plasticity of the material. The light-shielding material 131 may be non-deformable at the room temperature. For example, when the light-shielding material 131 is the glass or the plastic that does not have the plasticity at the room temperature, it is necessary to heat the light-shielding material 131 to soften the light-shielding material 131. And then the first light-transmission material 111, the second light-transmitting material 121, and the light-shielding material 131 are combined by squeezing. In this case, squeezing the first light-transmitting material 111, the second light-transmitting material 121, and the light-shielding material 131, to obtain a composite rod material 170, may include: after heating the first light-transmitting material 111, the second light-transmitting material 121, and the light-shielding material 131, squeezing the first light-transmitting material 111, the second light-transmitting material 121, and the light-shielding material 131, to obtain the composite rod material 170. Before squeezing, the first light-transmitting material 111 and the second light-transmitting material 121 may or may not have plasticity.

In some embodiments, a percentage difference between a thermal expansion coefficient (CTE) of the light-shielding material 131 and a thermal expansion coefficient of the first light-transmitting material 111 is less than or equal to 20%. The thermal expansion coefficient of the light-shielding material 131 needs to be as close as possible to the thermal expansion coefficient of the first light-transmitting material 111, which avoids or reduces a phenomenon of internal cracking of the light-shielding material 131 and/or the first light-transmitting material 111 caused by excessive CTE difference after high-temperature treating and cooling. For example, the percentage difference between the thermal expansion coefficient of the light-shielding material 131 and the thermal expansion coefficient of the first light-transmitting material 111 may be 20%, 15%, 10%, 5%, 4%, 3%, 2%, 1%, or 0. When the percentage difference between the thermal expansion coefficient of the light-shielding material 131 and the thermal expansion coefficient of the first light-transmitting material 111 is 0, the thermal expansion coefficient of the light-shielding material 131 is equal to the thermal expansion coefficient of the first light-transmitting material 111.

In some embodiments, the percentage difference between the thermal expansion coefficient of the light-shielding material 131 and a thermal expansion coefficient of the second light-transmitting material 121 is less than or equal to 20%. The thermal expansion coefficient of the light-shielding material 131 needs to be as close as possible to the thermal expansion coefficient of the second light-transmitting material 121, which avoids or reduces the phenomenon of internal cracking of the light-shielding material 131 and/or the second light-transmitting material 121 caused by the excessive CTE difference after high-temperature treating and cooling. For example, the percentage difference between the thermal expansion coefficient of the light-shielding material 131 and the thermal expansion coefficient of the second light-transmitting material 121 may be 20%, 15%, 10%, 5%, 4%, 3%, 2%, 1%, or 0. When the percentage difference between the thermal expansion coefficient of the light-shielding material 131 and the thermal expansion coefficient of the second light-transmitting material 121 is 0, the thermal expansion coefficient of the light-shielding material 131 is equal to the thermal expansion coefficient of the second light-transmitting material 121.

When a cross-sectional size of the composite rod material 170 that is obtained after squeezing is greater than a cross-sectional size of a target product (a cover plate), a step of pulling the composite rod material 170 may be provided after squeezing, to adjust the cross-sectional size of the composite rod material 170. And accordingly, cross-sectional sizes of a first window area 11, a second window area 12, and a light-shielding area 13 are simultaneously adjusted. Compared with the method of only squeezing, squeezing and pulling may quickly obtain the target product that meets the size requirements, thereby greatly improving the production efficiency and reducing the production cost. The bonding effect between the materials in the composite rod material 170 may be improved through pulling the composite rod material 170, so that the bonding between the materials of the composite rod material 170 is tighter.

From the perspective of improving the pulling effect, in some embodiments, a softening point temperature of the first light-transmitting material 111 is higher than or equal to a softening point temperature of the light-shielding material 131, and a softening point temperature of the second light-transmitting material 121 is higher than or equal to the softening point temperature of the light-shielding material 131. Otherwise, when the softening point temperature of the first light-transmitting material 111 or the softening point temperature of second light-transmitting material 121 is lower than the softening point temperature of the light-shielding material 131, during the process of heating the material before pulling, it is easy to occur a phenomenon that the light-shielding material 131 has not become soft and cannot be pulled, and the first light-transmitting material 111 or the second light-transmitting material 121 has been melted or softened, thereby causing inability to process. Therefore, in some embodiments, the softening point temperature of the light-shielding material 131, the softening point temperature of the first light-transmitting material 111, and the softening point temperature of the second light-transmitting material 121 are equal to each other.

When the first light-transmitting material 111, the second light-transmitting material 121, and the light-shielding material 131 are all softened in the composite rod material 170, the composite rod material 170 may be pulled. The cross-sectional size of each of the light-transmitting material 111, the second light-transmitting material 121, and the light-shielding material 131 may be simultaneously changed by pulling the composite rod material 170. When the first light-transmitting material 111 and the second light-transmitting material 121 are not softened, while the light-shielding material 131 has softened, the composite rod material 170 may also be pulled. In this case, the pulling is only for the light-shielding material 131, and only the cross-sectional size of the material 131 is changed by pulling, while the cross-sectional sizes of the first light-transmitting material 111 and the second light-transmitting material 121 are not changed.

In order to eliminate an internal stress generated during processing, and avoid or reduce the possibility of subsequent deformation and cracking of the composite rod material 170, in some embodiments, after squeezing, or squeezing and pulling, annealing the composite rod material 170 may be provided. An annealing temperature is determined according to specific properties of the material.

At block 140, the composite rod material is treated, to obtain the cover plate.

As illustrated in FIG. 4, FIG. 4 is a schematic process flow chart of processing the composite rod material prepared by the method as shown in FIG. 1 to obtain a cover plate. Treating the composite rod material may include: cutting the composite rod material 170. Of course, the treating the composite rod material may also be performed by other non-cutting methods. The composite rod material 170 may be treated according to a shape and a size of designed cover plate 10. The shape of the cover plate 10 may be circular, polygonal (such as triangular, quadrilateral, pentagonal, hexagonal), irregular, etc. After treating, subsequently processing the cover plate 10 may be performed, such as a computer numerical control (CNC) processing, a grinding, a polishing, a coating, a silk-screen printing, or other processes, so as to improve the quality of the cover plate 10.

As illustrated in FIG. 4, the cover plate 10 may include the first window area 11, the second window area 12, and the light-shielding area 13. The first window area 11 and the second window area 12 are spaced apart from each other, and the light-shielding area 13 surrounds the first window area 11 and surrounds the second window area 12.

The first window area 11 is formed by the first light-transmitting material 111, the second window area 12 is formed by the second light-transmitting material 121, and the light-shielding area 13 is formed by the light-shielding material 131.

When the cover plate 10 is applied to an electronic device with a photoplethysmographic (PPG) structure, both the first window area 11 and the second window area 12 should allow the light rays ranging from visible light to near infrared light (wavelength ranging from 400 nm to 1200 nm) to pass through. The higher the light transmittance of each of the first window area 11 and the second window area 12, the better. The lower the light transmittance of the light-shielding area 13 to the light rays ranging from the visible light to the near infrared light, the better.

In the above method for making the first type of cover plate, the first light-transmitting material 111, the second light-transmitting material 121, and the light-shielding material 131 are combined by squeezing, or by squeezing and pulling, so that the cover plate 10 is obtained. Both the first window area 11 and the second window area 12 in the cover plate 10 are surrounded by the light-shielding area 13, which may avoid optical crosstalk between the first window area 11 and the second window area 12.

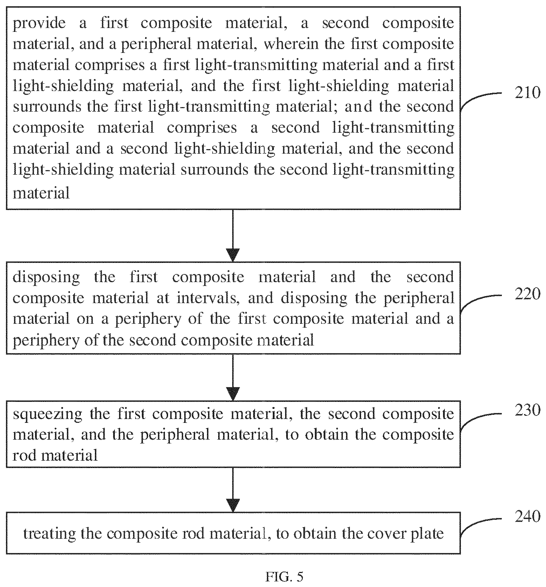

As illustrated in FIG. 5, FIG. 5 is a flow chart of a method for making a second type of cover plate in some embodiments of the present disclosure. The method for making the second type of cover plate includes the following operations.

At block 210, a first composite material, a second composite material, and a peripheral material are provided; the first composite material includes the first light-transmitting material and a first light-shielding material surrounding the first light-transmitting material, and the second composite material includes the second light-transmitting material and a second light-shielding material surrounding the second light-transmitting material.

Each of the first light-transmitting material and the second light-transmitting material may be selected from at least one of the transparent glass, the transparent plastic (such as PMMA), the transparent ceramic, and the sapphire. The light transmittance of the first light-transmitting material to the light rays of 400 nm-1200 nm is greater than or equal to 20%, such as 20%, 30%, 40%, 50%, 60%, 70%, 80%, 90%, or 100%. The light transmittance of the second light-transmitting material to the light rays of 400 nm-1200 nm is greater than or equal to 20%, such as 20%, 30%, 40%, 50%, 60%, 70%, 80%, 90%, or 100%.

Each of the first light-shielding material and the second light-shielding material may be selected from at least one of the opaque glass, the opaque plastic, the opaque ceramics, and the metal. The opaque plastic may be PC, PA, PEEK, etc., and the color of "opaque" may be black or dark. Both the first light-shielding material and the second light-shielding material may be black glass material. The black glass material may be an ordinary black glass material that is obtained by adding color powders to the transparent glass, or may be a black glass ceramic.

The light transmittance of the first light-shielding material to the light rays of 400 nm-1200 nm is less than or equal to 10%, such as 10%, 9%, 8%, 7%, 6%, 5%, 4%, 3%, 2%, 1%, or 0. The light transmittance of the second light-shielding material to the light rays of 400 nm-1200 nm is less than or equal to 10%, such as 10%, 9%, 8%, 7%, 6%, 5%, 4%, 3%, 2%, 1%, or 0.

The first light-transmitting material and the second light-transmitting material may be the same material or different materials. The first light-shielding material and the second light-shielding material may be the same material or different materials.

The peripheral material may be the glass, the plastic, the ceramic, the metal, etc., and a color of the peripheral material may be transparent, dark, or black.

At block 220, the first composite material and the second composite material are disposed at intervals, and the peripheral material is disposed on a periphery of the first composite material and a periphery of the second composite material.

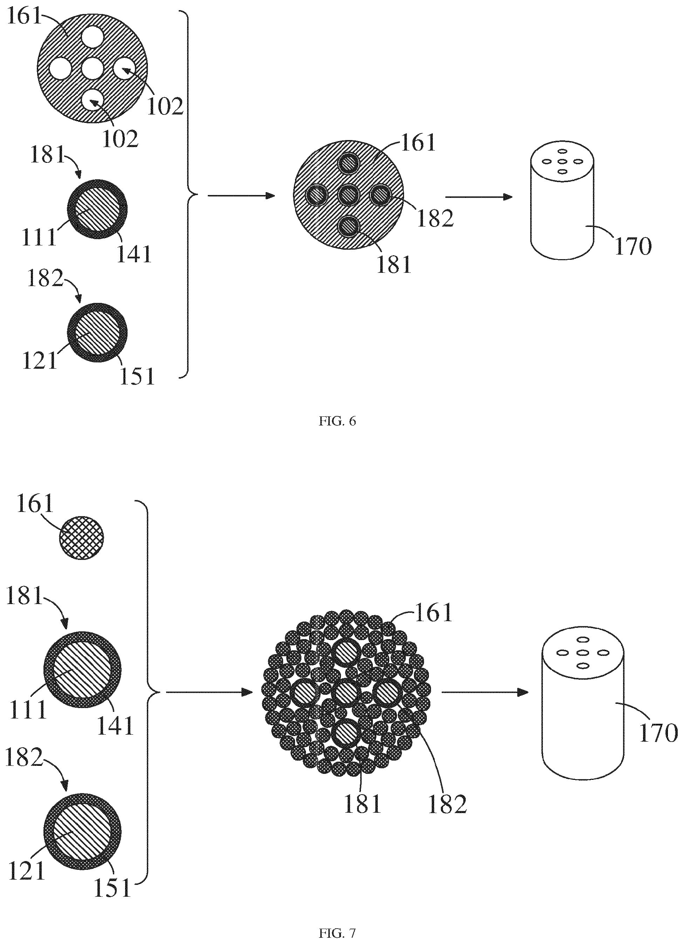

As illustrated in FIG. 6, FIG. 6 is a schematic process flow chart of a first type of method for making the composite rod material in the method shown in FIG. 5. A peripheral material 161 may be a single rod material, and the peripheral material 161 defines a plurality of first hollow structures 102 disposed at intervals. In this case, disposing the peripheral material on the periphery of the first composite material and the periphery of the second composite material, may include: disposing a first composite material 181 and a second composite material 182 in different first hollow structures 102, respectively. Each of the first hollow structures 102 may be the through hole or the groove. The number of the first hollow structures 102 in the peripheral material 161 is at least 2, and may be more than 2, such as 3, 4, 5, 6, 7, 8, etc.

As illustrated in FIG. 7, FIG. 7 is a schematic process flow chart of a second type of method for making the composite rod material in the method shown in FIG. 5. The peripheral material 161 may include the plurality of fiber filaments. In this case, disposing the peripheral material on the periphery of the first composite material and the periphery of the second composite material, may include: arranging the plurality of fiber filaments on the periphery of the first composite material 181 and the periphery of the second composite material 182. When the peripheral material 161 adopts the single rod material with an integral structure, it is necessary to punch holes on the peripheral material 161 to form the first hollow structures 102. For the materials that are difficult to process, such as the glass, there are often problems such as difficulty in drilling and cracking of the rod material, thereby increasing the processing difficulty. The peripheral material 161 adopts the plurality of fiber filaments, which avoids drilling, thereby reducing the processing difficulty, improving the production yield, and reducing the production cost.

The first composite material 181 may include a first composite rod body formed by combining the first light-transmitting material 111 and a first light-shielding material 141. In this case, providing the first composite material 181 may include the following operations:
providing the first light-transmitting material 111 and the first light-shielding material 141, and disposing the first light-shielding material 141 on the periphery of the first light-transmitting material 111; and
squeezing the first light-transmitting material 111 and the first light-shielding material 141, to obtain the first composite rod body.

By combining the first light-transmitting material 111 and the first light-shielding material 141 to form the first composite rod body, subsequently squeezing the first composite material 181 and the peripheral material 161 may improve the bonding effect between the first light-transmitting material 111, the first light-shielding material 141, and the peripheral material 161, thereby improving the production yield.

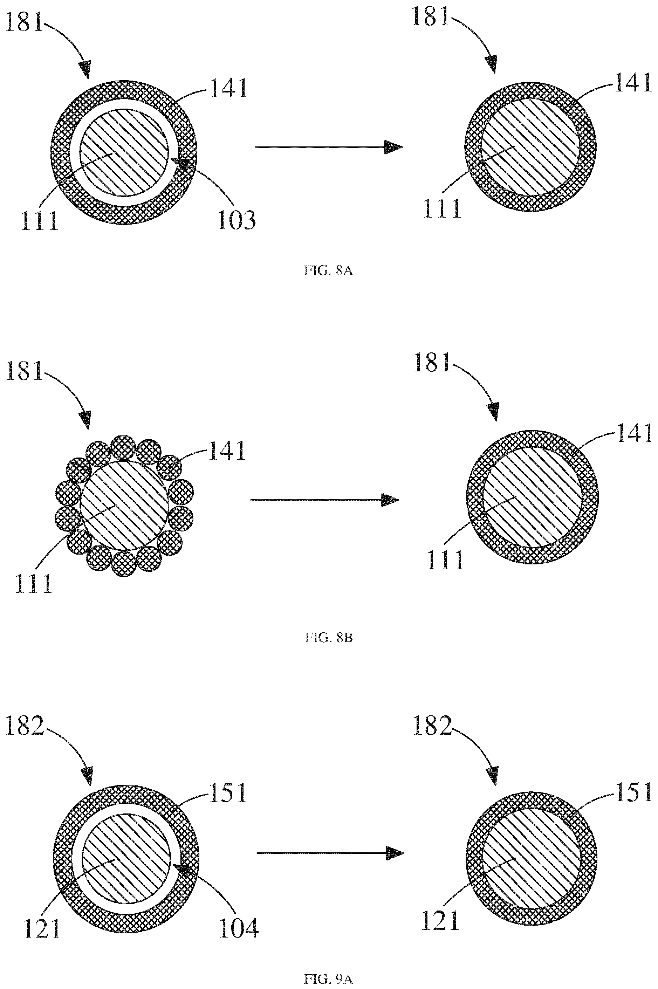

As illustrated in FIG. 8A, FIG. 8A is a schematic process flow chart of a first type of method for making a first composite rod body in the method shown in FIG. 5. The first light-shielding material 141 may be a single first opaque rod material, and the first light-shielding material 141 defines a second hollow structure 103. In this case, disposing the first light-shielding material 141 on the periphery of the first light-transmitting material 111, may include: disposing the first light-transmitting material 111 in the second hollow structure 103. The second hollow structure 103 may be the through hole or the groove. A size of the second hollow structure 103 needs to be greater than or equal to a size of the first light-transmitting material 111, so that the first light-transmitting material 111 may be inserted into the second hollow structure 103. In some embodiments, after inserting the first light-transmitting material 111 into the second hollow structure 103, a distance between an outer surface of the first light-transmitting material 111 and an inner surface of the second hollow structure 103 ranges from 0.3 mm to 1 mm. A cross-sectional shape of the first opaque rod material is designed according to specific requirements. The cross-sectional shape of the first opaque rod material may be circle or polygon, and the polygon may be quadrilateral (such as square, rectangle), pentagon, hexagon, heptagon, octagon, etc.

As illustrated in FIG. 8B, FIG. 8B is a schematic process flow chart of a second type of method for making a first composite rod body in the method shown in FIG. 5. The first light-shielding material 141 may include a plurality of first opaque fiber filaments. In this case, disposing the first light-shielding material 141 on the periphery of the first light-transmitting material 111 may include: arranging the plurality of first opaque fiber filaments on the periphery of the first light-transmitting material 111.

The first light-shielding material 141 may be deformed under the action of the external force and at the room temperature. For example, when the first light-shielding material 141 is the metal or the plastic that has plasticity at the room temperature, the first light-transmitting material 111 and the first light-shielding material 141 may be combined by only squeezing without heating. Of course, the first light-transmitting material 111 and the first light-shielding material 141 may also be heated to improve the plasticity of the material. The first light-shielding material 141 may be non-deformable at the room temperature. For example, when the first light-shielding material 141 is the glass or the plastic that does not have plasticity at the room temperature, it is necessary to heat the first light-shielding material 141 to soften the first light-shielding material 141. And then the first light-shielding material 111 and the first light-shielding material 141 may be combined by squeezing. In this case, squeezing the first light-transmitting material 111 and the first light-shielding material 141 to obtain the first composite rod body may include: after heating the first light-transmitting material 111 and the first light-shielding material 141, squeezing the first light-transmitting material 111 and the first light-shielding material 141, to obtain the first composite rod body. Before squeezing, the first light-transmitting material 111 may or may not have plasticity.

In some embodiments, a percentage difference between the thermal expansion coefficient of the first light-transmitting material 111 and a thermal expansion coefficient of the first light-shielding material 141 is less than or equal to 20%. A definition of "percentage difference" is: if A is greater than or equal to B, a percentage difference between A and B = (A-B)/B. All "percentage difference" recited in the present disclosure adopts this definition. The thermal expansion coefficient of the light-shielding material 131 needs to be as close as possible to the thermal expansion coefficient of the first light-shielding material 141, which avoids or reduces the phenomenon of internal cracking of the first light-transmitting material 111 and/or the first light-shielding material 141 caused by the excessive CTE difference after the high-temperature treating and cooling. In some embodiments, the percentage difference between the thermal expansion coefficient of the first light-transmitting material 111 and the thermal expansion coefficient of the first light-shielding material 141 may be 20%, 15%, 10%, 5%, 4%, 3%, 2%, 1 %, or 0. When the percentage difference between the thermal expansion coefficient of the first light-transmitting material 111 and the thermal expansion coefficient of the first light-shielding material 141 is 0, the thermal expansion coefficient of the first light-transmitting material 111 is equal to the thermal expansion coefficient of the first light-shielding material 141.

When a cross-sectional size and a length of the first composite rod body need to be changed, a step of pulling the first composite rod body may be provided after squeezing. From the perspective of improving the pulling effect, in some embodiments, the softening point temperature of the first light-transmitting material 111 is higher than or equal to a softening point temperature of the first light-shielding material 141. Otherwise, when the softening point temperature of the first light-transmitting material 111 is lower than the softening point of the first light-shielding material 141, during the process of heating the material before pulling, it is easy to occur a phenomenon that the first light-shielding material 141 has not become soft and cannot be pulled, and the first light-transmitting material 111 has been melted or softened, thereby causing inability to process. In some embodiments, the softening point temperature of the first light-shielding material 141 is equal to the softening point temperature of the first light-transmitting material 111.

When both the first light-transmitting material 111 and the first light-shielding material 141 are softened in the first composite rod body, the first composite rod body may be pulled. The cross-sectional sizes of the first light-transmitting material 111 and the first light-shielding material 141 may be simultaneously changed by pulling the first composite rod body. When the first light-transmitting material 111 is not softened, while the first light-shielding material 141 has been softened, the first composite rod body may also be pulled. In this case, the pulling is only for the first light-shielding material 141, and only the cross-sectional size of the first light-shielding material 141 is changed by pulling, while the cross-sectional size of the first light-transmitting material 111 is not changed.

In order to eliminate the internal stress generated during processing, and avoid or reduce the possibility of subsequent deformation and cracking of the first composite rod body, in some embodiments, after squeezing, or after squeezing and pulling, annealing the first composite rod body may be provided. An annealing temperature is determined according to the specific properties of the material.

The second composite material 182 may include a second composite rod body formed by combining the second light-transmitting material 121 and a second light-shielding material 151. In this case, providing the second composite material 182 may include the following operations:
providing the second light-transmitting material 121 and the second light-shielding material 151, and disposing the second light-shielding material 151 on a periphery of the second light-shielding material 121; and
squeezing the second light-transmitting material 121 and the second light-shielding material 151, to obtain the second composite rod body.

By combining the second light-transmitting material 121 and the second light-shielding material 151 to form the second composite rod body, subsequently squeezing the second composite material 182 and the peripheral material 161 may improve the bonding effect between the second light-transmitting material 121, the second light-shielding material 151, and the peripheral material 161, thereby improving the production yield.

As illustrated in FIG. 9A, FIG. 9A is a schematic process flow chart of a first type of method for making a second composite rod body in the method shown in FIG. 5. The second light-shielding material 151 may be a single second opaque rod material. The second light-shielding material 151 defines a third hollow structure 104. In this case, disposing the second light-shielding material 151 on the periphery of the second light-transmitting material 121, may include: disposing the second light-transmitting material 121 in the third hollow structure 104. The third hollow structure 104 may be the through hole or the groove. A size of the third hollow structure 104 needs to be greater than or equal to a size of the second light-transmitting material 121, so that the second light-transmitting material 121 may be inserted into the third hollow structure 104. In some embodiments, after inserting the second light-transmitting material 121 into the third hollow structure 104, a distance between an outer surface of the second light-transmitting material 121 and an inner surface of the third hollow structure 104 ranges from 0.3 mm to 1 mm. A cross-sectional shape of the second opaque rod material may be designed according to specific requirements. The cross-sectional shape of the second opaque rod material may be circle or polygon, and the polygon may be quadrilateral (such as square, rectangle), pentagon, hexagon, heptagon, octagon, etc.

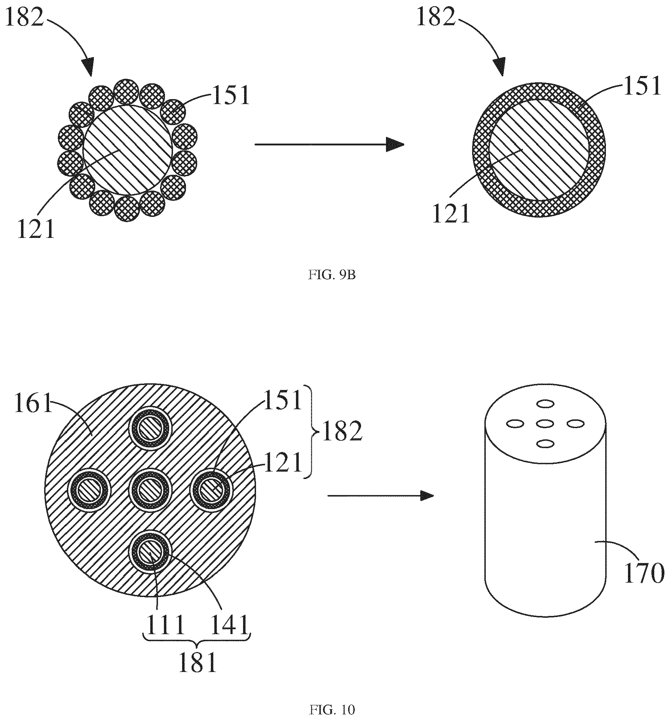

As illustrated in FIG. 9B, FIG. 9B is a schematic process flow chart of a second type of method for making a second composite rod body in the method shown in FIG. 5. The second light-shielding material 151 may also include a plurality of second opaque fiber filaments. In this case, disposing the second light-shielding material 151 on the periphery of the second light-transmitting material 121, may include: arranging the plurality of second opaque fiber filaments on the periphery of the second light-transmitting material 121.

The second light-shielding material 151 may be deformed under the action of the external force and at the room temperature. For example, when the second light-shielding material 151 is the metal or the plastic that has plasticity at the room temperature, the second light-transmitting material 121 and the second light-shielding material 151 may be combined by only squeezing without heating. Of course, the second light-transmitting material 121 and the second light-shielding material 151 may also be heated to improve the plasticity of the material. The second light-shielding material 151 may be non-deformable at the room temperature. For example, when the second light-shielding material 151 is the glass or the plastic that does not have the plasticity at the room temperature, it is necessary to heat the second light-shielding material 151 to soften the second light-shielding material 151. And then the second light-shielding material 121 and the second light-shielding material 151 may be combined by squeezing. In this case, squeezing the second light-transmitting material 121 and the second light-shielding material 151 to obtain the second composite rod body, may include: after heating the second light-transmitting material 121 and the second light-shielding material 151, squeezing the second light-transmitting material 121 and the second light-shielding material 151, to obtain the second composite rod body. Before squeezing, the second light-transmitting material 121 may or may not have plasticity.

In some embodiments, a percentage difference between the thermal expansion coefficient of the second light-transmitting material 121 and a thermal expansion coefficient of the second light-shielding material 151 is less than or equal to 20%. The thermal expansion coefficient of the second light-transmitting material 121 needs to be as close as possible to the thermal expansion coefficient of the second light-shielding material 151, which avoids or reduces the phenomenon of internal cracking of the second light-transmitting material 121 and/or the second light-shielding material 151 caused by the excessive CTE difference after the high-temperature treating and cooling. In some embodiments, the percentage difference between the thermal expansion coefficient of the second light-transmitting material 121 and the thermal expansion coefficient of the second light-shielding material 151 may be 20%, 15%, 10%, 5%, 4%, 3%, 2%, 1 %, or 0. When the percentage difference between the thermal expansion coefficient of the second light-transmitting material 121 and the thermal expansion coefficient of the second light-shielding material 151 is 0, the thermal expansion coefficient of the second light-transmitting material 121 is equal to the thermal expansion coefficient of the second light-shielding material 151.

When a cross-sectional size and a length of the second composite rod body need to be changed, a step of pulling the second composite rod body may be provided after squeezing. From the perspective of improving the pulling effect, in some embodiments, the softening point temperature of the second light-transmitting material 121 is higher than or equal to a softening point temperature of the second light-shielding material 151. Otherwise, when the softening point temperature of the second light-transmitting material 121 is lower than the softening point of the second light-shielding material 151, during the process of heating the material before pulling, it is easy to occur a phenomenon that the second light-shielding material 151 has not become soft and cannot be pulled, while the second light-transmitting material 121 has been melted or softened, thereby causing inability to process. In some embodiments, the softening point temperature of the second light-shielding material 151 is equal to the softening point temperature of the second light-transmitting material 121.

When both the second light-transmitting material 121 and the second light-shielding material 151 are softened in the second composite rod body, the second composite rod body may be pulled. The cross-sectional sizes of the second light-transmitting material 121 and the second light-shielding material 151 may be simultaneously changed by pulling the second composite rod body. When the second light-transmitting material 121 is not softened, while the second light-shielding material 151 has been softened, the second composite rod body may also be pulled. In this case, the pulling is only for the second light-shielding material 151, and only the cross-sectional size of the second light-shielding material 151 may be changed by pulling, while the cross-sectional size of the second light-transmitting material 121 is not changed.

In order to eliminate the internal stress generated during processing, and avoid or reduce the possibility of subsequent deformation and cracking of the second composite rod body, in some embodiments, after squeezing, or after squeezing and pulling, annealing the second composite rod body may be provided. An annealing temperature is determined according to the specific properties of the material.

As illustrated in FIG. 10, FIG. 10 is a schematic process flow chart of a third type of method for making the composite rod material in the method shown in FIG. 5. In the first composite material 181, there may also be a gap between the first light-transmitting material 111 and the first light-shielding material 141. That is, the first light-transmitting material 111 and the first light-shielding material 141 are spaced apart from each other and not combined together. The step of forming the first composite rod body may be omitted, so that one production process may be omitted, thereby improving production efficiency and reducing production cost.

As illustrated in FIG. 10, in the second composite material 182, there may also be a gap between the second light-transmitting material 121 and the second light-shielding material 151. That is, the second light-transmitting material 121 and the second light-shielding material 151 are spaced apart from each other and not combined together. The step of forming the second composite rod body may be omitted, so that one production process may be omitted, thereby improving production efficiency and reducing production cost.

The first light-transmitting material 111 may be the single first light-transmitting rod material, or the first light-transmitting material 111 includes the plurality of first light-transmitting fiber filaments. The second light-transmitting material 121 may be the single second light-transmitting rod material, or the second light-transmitting material 121 includes the plurality of second light-transmitting fiber filaments.

At block 230, the first composite material, the second composite material, and the peripheral material are squeezed, to obtain the composite rod material.

As illustrated in FIG. 6, FIG. 7, or FIG. 10, the peripheral material 161 may be deformed under the action of the external force and at the room temperature. For example, when the peripheral material 161 is the metal or the plastic that has plasticity at the room temperature, the first composite material 181, the second composite material 182, and the peripheral material 161 may be combined by only squeezing without heating. Of course, the first composite material 181, the second composite material 182, and the peripheral material 161 may also be heated, to improve the plasticity of the material. The peripheral material 161 may be non-deformable at the room temperature, for example, when the peripheral material 161 is the glass or the plastic that does not have plasticity at the room temperature, it is necessary to heat the peripheral material 161 to soften the peripheral material 161. Ane then the first composite material 181, the second composite material 182, and the peripheral material 161 may be combined by squeezing. In this case, squeezing the first composite material 181, the second composite material 182, and the peripheral material 161 to obtain the composite rod material 170, may include: after heating the first composite material 181, the second composite material 182, and the peripheral material 161, squeezing the first composite material 181, the second composite material 182, and the peripheral material 161, to obtain the composite rod material 170. Before squeezing, the first composite material 181 and the second composite material 182 may or may not have plasticity.

In some embodiments, in the first light-transmitting material 111, the first light-shielding material 141, and the peripheral material 161, a percentage difference between thermal expansion coefficients of any two is less than or equal to 20%. A thermal expansion coefficient of the peripheral material 161 needs to be as close as possible to the thermal expansion coefficient of the first light-transmitting material 111 and the thermal expansion coefficient of the first light-shielding material 141, which avoids or reduces the phenomenon of internal cracking of the first light-transmitting material 111 and/or the first light-shielding material 141 and/or the peripheral material 161 caused by the excessive CTE difference after the high-temperature treating and cooling. In some embodiments, in the first light-transmitting material 111, the first light-shielding material 141, and the peripheral material 161, the percentage difference between the thermal expansion coefficients of any two may be 20%, 15%, 10%, 5%, 4%, or 3%. %, 2%, 1%, or 0. When the percentage difference between the thermal expansion coefficients of any two of the first light-transmitting material 111, the first light-shielding material 141, and the peripheral material 161 is 0, the thermal expansion coefficient of the first light-transmitting material 111, the thermal expansion coefficient of the first light-shielding material 141, and the thermal expansion coefficient of the peripheral material 161 are equal to each other.

In some embodiments, in the second light-transmitting material 121, the second light-shielding material 151, and the peripheral material 161, a percentage difference between the thermal expansion coefficients of any two is less than or equal to 20%. The thermal expansion coefficient of the peripheral material 161 needs to be as close as possible to the thermal expansion coefficient of the second light-transmitting material 121 and the thermal expansion coefficient of the second light-shielding material 151, which avoids or reduces the phenomenon of internal cracking of the second light-transmitting material 121 and/or the second light-shielding material 151 and/or the peripheral material 161 caused by the excessive CTE difference after the high-temperature treating and cooling. In some embodiments, in the second light-transmitting material 121, the second light-shielding material 151, and the peripheral material 161, the percentage difference between the thermal expansion coefficients of any two may be 20%, 15%, 10%, 5%, 4%, or 3%. %, 2%, 1%, or 0. When the percentage difference between the thermal expansion coefficients of any two of the second light-transmitting material 121, the second light-shielding material 151, and the peripheral material 161 is 0, the thermal expansion coefficient of the second light-transmitting material 121, the thermal expansion coefficient of the second light-shielding material 151, and the thermal expansion coefficient of the peripheral material 161 are equal to each other.

In some embodiments, a difference between the thermal expansion coefficient of the first light-transmitting material 111 and the thermal expansion coefficient of the peripheral material 161 is defined as a first difference. A difference between the thermal expansion coefficient of the first light-transmitting material 111 and the thermal expansion coefficient of the first light-shielding material 141 is defined as a second difference. An absolute value of the first difference is less than or equal to an absolute value of the second difference. Since an area formed by the peripheral material 161 is a non-working area, there is no requirement for the light transmittance, the selection range of the peripheral material 161 is relatively wide. By selecting the peripheral material 161 with the thermal expansion coefficient closer to that of the first light-transmitting material 111, the phenomenon of the internal cracking of the first light-transmitting material 111 caused by excessive CTE difference after the high-temperature treating and cooling may be avoided or reduced.

In some embodiments, a difference between the thermal expansion coefficient of the second light-transmitting material 121 and the thermal expansion coefficient of the peripheral material 161 is defined as a third difference. A difference between the thermal expansion coefficient of the second light-transmitting material 121 and the second light-shielding material 151 is defined as a fourth difference. An absolute value of the third difference is less than or equal to an absolute value of the fourth difference. By selecting the peripheral material 161 with the thermal expansion coefficient closer to that of the second light-transmitting material 121, the phenomenon of the internal cracking of the second light-transmitting material 121 caused by excessive CTE difference after the high-temperature treating and cooling may be avoided or reduced.

In some embodiments, the first light-transmitting material 111, the second light-transmitting material 121, and the peripheral material 161 are the same material. That is, the first light-transmitting material 111, the second light-transmitting material 121, and the peripheral material 161 are all light-transmitting materials. In this way, the thermal expansion coefficients of the first light-transmitting material 111, the second light-transmitting material 121, and the peripheral material 161 are equal to each other. After squeezing and cooling, the first light-transmitting material 111 and the second light-transmitting material 121 are not easy to crack.

When the cross-sectional size of the composite rod material 170 that is obtained after squeezing is greater than the cross-sectional size of the target product (the cover plate), the step of pulling the composite rod material 170 may be provided after squeezing, to adjust the cross-sectional size of the composite rod material 170. And accordingly, the cross-sectional sizes of the first window area 11, the second window area 12, and the light-shielding area 13 are simultaneously adjusted. Compared with the method of only squeezing, squeezing and pulling may quickly obtain the target product that meets the size requirements, thereby greatly improving the production efficiency and reducing the production cost. The bonding effect between the materials in the composite rod material 170 may be improved through pulling the composite rod material 170, so that the bonding between the materials of the composite rod material 170 is tighter.

From the perspective of improving the pulling effect, in some embodiments, the softening point temperature of the first light-transmitting material 111 is higher than or equal to the softening point temperature of the first light-shielding material 141, and the softening point temperature of the first light-shielding material 141 is higher than or equal to a softening point temperature of the peripheral material 161. Otherwise, when the softening point temperature of the first light-shielding material 141 is lower than the softening point temperature of the peripheral material 161, during the process of heating the material before pulling, it is easy to occur a phenomenon that the peripheral material 161 has not become soft and cannot be pulled, while the first light-shielding material 141 has been melted or softened, thereby causing inability to process. When the softening point temperature of the first light-transmitting material 111 is lower than the softening point temperature of the first light-shielding material 141, during the process of heating the material before pulling, it is easy to occur the phenomenon that the first light-shielding material 141 that is located on the periphery has not become soft, while the first light-transmitting material 111 that is located inside has been melted or softened, thereby causing inability to process. In some embodiments, the softening point temperature of the first light-transmitting material 111, the softening point temperature of the first light-shielding material 141, and the softening point temperature of the peripheral material 161 are equal to each other.

From the perspective of improving the pulling effect, in some embodiments, the softening point temperature of the second light-transmitting material 121 is higher than or equal to the softening point temperature of the second light-shielding material 151, and the softening point temperature of the second light-shielding material 151 is higher than or equal to the softening point temperature of the peripheral material 161. Otherwise, when the softening point temperature of the second light-shielding material 151 is lower than the softening point temperature of the peripheral material 161, during the process of heating the material before pulling, it is easy to occur a phenomenon that the peripheral material 161 has not become soft and cannot be pulled, while the second light-shielding material 151 has been melted or softened, thereby causing inability to process. When the softening point temperature of the second light-transmitting material 121 is lower than the softening point temperature of the second light-shielding material 151, during the process of heating the material before pulling, it is easy to occur the phenomenon that the second light-shielding material 151 that is located on the periphery has not become soft and cannot be pulled, while the second light-transmitting material 121 that is located inside has been melted or softened, thereby causing inability to process. In some embodiments, the softening point temperature of the second light-transmitting material 121, the softening point temperature of the second light-shielding material 151, and the softening point temperature of the peripheral material 161 are equal to each other.

When the first light-transmitting material 111, the second light-transmitting material 121, the first light-shielding material 141, the second light-shielding material 151, and the peripheral material 161 are all softened in the composite rod material 170, the composite rod material 170 may be pulled. The cross-sectional size of each of the first light-transmitting material 111, the second light-transmitting material 121, the first light-shielding material 141, the second light-shielding material 151, and the peripheral material 161 may be simultaneously changed by pulling the composite rod material 170. When the first light-transmitting material 111 and the second light-transmitting material 121 are not softened, while the first light-shielding material 141, the second light-shielding material 151, and the peripheral material 161 have been softened, the composite rod material 170 may also be pulled. In this case, the pulling is only for the first light-shielding material 141, the second light-shielding material 151, and the peripheral material 161. The cross-sectional sizes of the first light-shielding material 141, the second light-shielding material 151, and the peripheral material 161 are changed by pulling, while the cross-sectional sizes of the first light-transmitting material 111 and the second light-transmitting material 121 are not changed. When the first light-transmitting material 111, the second light-transmitting material 121, the first light-shielding material 141, and the second light-shielding material 151 are not softened, while the peripheral material 161 has been softened, the composite rod material 170 may also be pulled. In this case, the pulling is only for the peripheral material 161, and only the cross-sectional size of the peripheral material 161 is changed, while the cross-sectional sizes of the first light-transmitting material 111, the second light-transmitting material 121, the first light-shielding material 141, and the second light-shielding material 151 are not changed.

In order to eliminate the internal stress generated during processing, and avoid or reduce the possibility of subsequent deformation and cracking of the composite rod material 170, in some embodiments, after squeezing, or after squeezing and pulling, annealing the composite rod material 170 may be provided. The annealing temperature is determined according to the specific properties of the material.

At block 240, the composite rod material is treated, to obtain the cover plate.

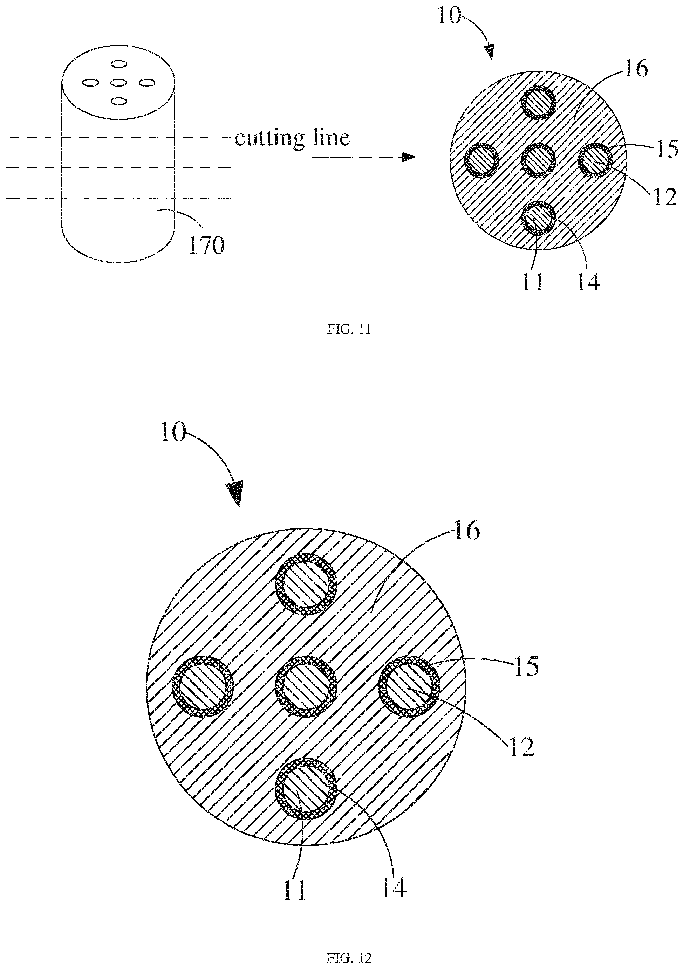

As illustrated in FIG, 11, FIG. 11 is a schematic process flow chart of processing the composite rod material prepared by the method as shown in FIG. 5 to obtain the cover plate. Treating the composite rod material may include: cutting the composite rod material 170. Of course, the treating the composite rod material may also be performed by other non-cutting methods. The composite rod material 170 may be treated according to the shape and the size of the designed cover plate 10. The shape of the cover plate 10 may be circular, polygonal (such as triangular, quadrilateral, pentagonal, hexagonal), irregular, etc. After treating, subsequently processing the cover plate 10 may be performed, such as the CNC processing, the grinding, the polishing, the coating, the silk-screen printing, etc., so as to improve the quality of the cover plate 10.

As illustrated in FIG, 11, the cover plate 10 may include the first window area 11, the second window area 12, a first light-shielding area 14, a second light-shielding area 15, and a peripheral area 16. The first window area 11 and the second window area 12 are disposed at intervals. The first light-shielding area 14 surrounds the first window area 11, and the second light-shielding area 15 surrounds the second window area 12. The peripheral area 16 surrounds the first light-shielding area 14 and the second light-shielding area 15. The first window area 11 is formed by the first light-transmitting material 111, and the second window area 12 is formed by the second light-transmitting material 121. The first light-shielding area 14 is formed by the first light-shielding material 141, and the second light-shielding area 15 is formed by the second light-shielding material 151. The peripheral area 16 is formed by the peripheral material 161.

In the above method for making the second type of cover plate, the first light-transmitting material 111, the second light-transmitting material 121, the first light-shielding material 141, the second light-shielding material 151, and the peripheral material 161 are combined by squeezing or by squeezing and pulling, so that the cover plate 10 is obtained. In the cover plate 10, the first light-shielding area 14 is disposed on a periphery of the first window area 11, and the second light-shielding area 15 is disposed on a periphery of the second window area 12, which may avoid the optical crosstalk between the first window area 11 and the second window area 12.

In some embodiments of the present disclosure, the cover plate is provided. As illustrated in FIG. 12, FIG. 12 is a structural schematic view of the cover plate in some embodiments of the present disclosure. The cover plate 10 may include the first window area 11, the second window area 12, the first light-shielding area 14, the second light-shielding area 15, and the peripheral area 16. The first window area 11 and the second window area 12 are configured to allow the light rays to pass through. The first light-shielding area 14 and the second light-shielding area 15 are configured to shield the light rays. The first window area 11 and the second window area 12 are disposed at intervals. The first light-shielding area 14 surrounds the first window area 11, and the second light-shielding area 15 surrounds the second window area 12, and the peripheral area 16 surrounds the first light-shielding area 14 and the second light-shielding area 15. The cover plate 10 may be prepared by the method for making the cover plate that is shown in FIG. 5, and of course, the cover plate 10 may also be prepared by other methods.

In some embodiments, a difference between a thermal expansion coefficient of a material of the first window area 11 and a thermal expansion coefficient of a material of the peripheral area 16 is defined as the first difference. A difference between the thermal expansion coefficient of the material of the first window area 11 and a thermal expansion coefficient of a material of the first light-shielding area 14 is defined as a second difference. The absolute value of the first difference is less than or equal to the absolute value of the second difference. The cover plate 10 is prepared according to the method for making the cover plate that is shown in FIG. 5, which may avoid or reduce the occurrence of cracking in the first window area 11.

In some embodiments, a difference between a thermal expansion coefficient of a material of the second window area 12 and the thermal expansion coefficient of the material of the peripheral area 16 is defined as the third difference. A difference between the thermal expansion coefficient of the material of the second window area 12 and a thermal expansion coefficient of a material of the second light-shielding area 15 is defined as the fourth difference. The absolute value of the third difference is less than or equal to the absolute value of the fourth difference. The cover plate 10 is prepared according to the method for making the cover plate that is shown in FIG. 5, which may avoid or reduce the occurrence of cracking in the second window area 12.

The material of the peripheral area 16 may be a light-transmitting material or an opaque material. In some embodiments, a light transmittance of the light-transmitting material to the light rays of 400 nm-1200 nm is greater than or equal to 20%, such as 20%, 30%, 40%, 50%, 60%, 70%, 80%, 90%, or 100%. A light transmittance of the opaque material to the light rays of 400 nm-1200 nm is less than or equal to 10%, such as 10%, 9%, 8%, 7%, 6%, 5%, 4%, 3%, 2%, 1 %, or 0. The peripheral area 16 is the non-working area, and there is no requirement for the light transmittance. Thus, a light transparency and a color of the peripheral area 16 may be adjusted according to specific requirements, so as to increase the richness of the appearance of the cover plate 10 and enhance the visual expression of the cover plate 10, thereby enhancing the visual expression and market competitiveness of the electronic device including the cover plate 10.

In some embodiments, the material of the first window area 11, the material of the second window area 12, and the material of the peripheral area 16 are the same material. That is, the material of the first window area 11, the material of the second window area 12, and the material of the peripheral area 16 are the light-transmitting materials. In this case, a proportion of the light-transmitting material in the cover plate 10 shown in FIG. 12 is relatively large. Compared with the cover plate 10 shown in FIG. 4, the proportion of the light-transmitting material in the cover plate 10 shown in FIG. 12 is significantly improved. In the case of a large difference in thermal expansion coefficients between the light-transmitting material and the opaque material, the first window area 11 and the second window area 12 in the cover plate 10 shown in FIG. 12 are relatively less prone to cracking. Certainly, the material of the first window area 11, the material of the second window area 12, and the material of the peripheral area 16 may also be different materials.

In some embodiments, each of the material of the first window area 11, the material of the second window area 12, and the material of the peripheral area 16 is a colorless transparent glass material. Each of the material of the first light-shielding area 14 and the material of the second light-shielding area 15 is the black glass material.

When the prepared cover plate 10 is applied to the electronic device with the PPG structure, both the first window area 11 and the second window area 12 should allow the light rays ranging from the visible light to the near infrared light (wavelength ranging from 400 nm to 1200 nm) to pass through. The higher the light transmittance of each of the first window area 11 and the second window area 12, the better. The lower the light transmittance of each of the first light-shielding area 14 and the second light-shielding area 15 to the light rays ranging from the visible light to the near-infrared light, the better.

The number of the first window area 11 provided on the cover plate 10 may be one or more, and the number of the second window area 12 provided on the cover plate 10 may be one or more.

The first light-shielding area 14 and the second light-shielding area 15 may be disposed at intervals or connected to each other.

In the cover plate 10 provided by the embodiments of the present disclosure, the first light-shielding area 14 is disposed on the periphery of the first window area 11, and the second light-shielding area 15 is disposed on the periphery of the second window area 12, which may avoid optical crosstalk between the first window area 11 and the second window area 12.

In some embodiments of the present disclosure, an electronic device is provided. The electronic device may be a wearable device, such as a watch, a bracelet, a ring, an arm cover, clothing, etc. In some embodiments, the electronic device has a health monitoring function. The health monitoring function includes, but not limited to, a blood oxygen monitoring function, a heart rate monitoring function, etc. In some embodiments, the health monitoring function of the electronic device is realized by photoplethysmography (PPG). The following electronic device being a smart watch with health monitoring function is taken as an example for illustration.

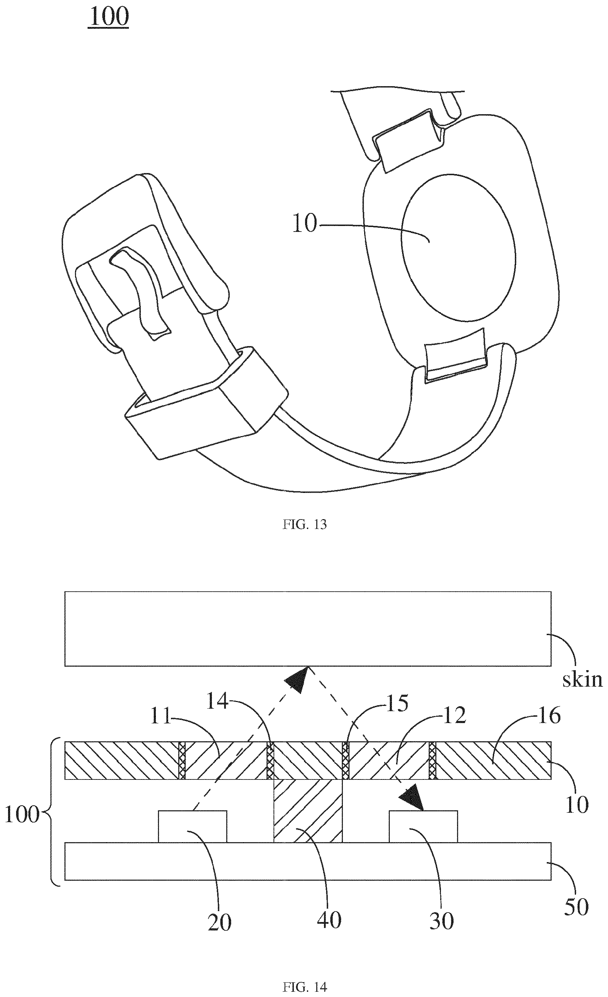

As illustrated in FIG. 13 and FIG. 14, FIG. 13 is a partial structural schematic view of an electronic device in some embodiments of the present disclosure. FIG. 14 is a partial structural schematic view of the electronic device shown in FIG. 13. An electronic device 100 may include: the cover plate 10, a light emitter 20 and a light detector 30. The light emitter 20 and the light detector 30 are located on the same side of the cover plate 10. The cover plate 10 is the cover plate 10 shown in FIG. 12. The light rays emitted by the light emitter 20 enter the first window area 11 and are transmitted to a side of the cover plate 10 away from the light emitter 20 through the first window area 11. The light rays that enter the second window area 12 from a side of the cover plate 10 away from the light detector 30 pass through the second window area 12 and enter the light detector 30. The cover plate 10 may be used as a back cover of the smart watch (that is, the cover plate 10 is close to a skin). The light emitter 20 may include an LED light, and the light detector 30 may include a light sensor.

The operating principle of the electronic device 100 is as follows. The light rays emitted by the light emitter 20 pass through the first window area 11 of the cover plate 10 and enter the skin. A part of the light rays is absorbed, another part of the light rays is reflected. The reflected light rays pass through the second window area 12 of the cover plate 10 to enter the light detector 30. Light signals received by the light detector 30 may be converted, to obtain relevant physiological information of the human body, such as the blood oxygen data, etc.

As illustrated in FIG. 14, the electronic device 100 may further include a circuit board 50. The circuit board 50 may be a printed circuit board (PCB), and the light emitter 20 and the light detector 30 are mounted on the circuit board 50. In addition, the electronic device 100 may also include an optical isolation structure 40. The optical isolation structure 40, the optical emitter 20, and the optical detector 30 are located on the same side of the cover plate 10. The optical isolation structure 40 is disposed between the optical emitter 20 and the optical detector 30, so as to prevent the light rays emitted by the optical emitter 20 from entering the optical detector 30. The optical isolation structure 40 may be disposed on the circuit board 50. In some embodiments, a top surface of the light isolation structure 40 abuts against a bottom surface of the cover plate 10, to maximum shielding the light rays emitted by the light emitter 20, thereby avoiding the optical crosstalk. A material of the light isolation structure 40 may be the opaque glass, the opaque ceramic, or the opaque plastic.

The electronic device 100 provided by the embodiments of the present disclosure adopts the above cover plate 10, which may avoid the light rays emitted by the light emitter 20 to pass through the second window area 12 from the first window area 11 without entering the skin, and then to be received by the optical detector 30. And accordingly, errors in the optical signals received by optical detector 30 may be avoided, thereby improving signal-to-noise ratios of the optical signals received by optical detector 30, and improving the accuracy of detection results.

The method for making the cover plate, the cover plate, and the electronic device provided in the embodiments of the present disclosure are described in detail above. In the present disclosure, some or embodiments or examples are used to illustrate the principles and implementation methods of the present disclosure. The descriptions of the above embodiments are only used to help understand the present disclosure. Furthermore, for those of ordinary skill in the art, based on the idea of the present disclosure, there will be changes in the specific implementation and application scope. In summary, the contents of the present specification should not be construed as limiting the disclosure.

## Claims

1. A method for making a cover plate (10), comprising:
providing (210) a first composite material (181), a second composite material (182), and a peripheral material (161), wherein the first composite material (181) comprises a first light-transmitting material (111) and a first light-shielding material (141), and the first light-shielding material (141) surrounds the first light-transmitting material (111); and the second composite material (182) comprises a second light-transmitting material (121) and a second light-shielding material (151), and the second light-shielding material (151) surrounds the second light-transmitting material (121);
disposing (220) the first composite material (181) and the second composite material (182) at intervals, and disposing the peripheral material (161) on a periphery of the first composite material (181) and a periphery of the second composite material (182);
**characterized in that** the method for making the cover plate (10) further comprises squeezing (230) the first composite material (181), the second composite material (182), and the peripheral material (161), to obtain a composite rod material (170); and
treating (240) the composite rod material (170) to obtain a cover plate (10).

2. The method as claimed in claim 1, wherein the peripheral material (161) is a single rod material, and the peripheral material (161) defines a plurality of first hollow structures (102) disposed at intervals; the disposing the peripheral material (161) on a periphery of the first composite material (181) and a periphery of the second composite material (182), comprises: disposing the first composite material (181) and the second composite material (182) in different first hollow structures (102) respectively; or
the peripheral material (161) comprises a plurality of fiber filaments; the disposing the peripheral material (161) on a periphery of the first composite material (181) and a periphery of the second composite material (182), comprises: disposing the plurality of the fiber filaments on the periphery of the first composite material (181) and the periphery of the second composite material (182).

3. The method as claimed in claim 1, wherein the first composite material (181) comprises a first composite rod body formed by combining the first light-transmitting material (111) and the first light-shielding material (141); and/or
the second composite material (182) comprises a second composite rod body formed by combining the second light-transmitting material (121) and the second light-shielding material (151).

4. The method as claimed in claim 3, wherein
the providing (210) a first composite material (181) comprises:
providing the first light-transmitting material (111) and the first light-shielding material (141), and disposing the first light-shielding material (141) on a periphery of the first light-transmitting material (111); and
squeezing the first light-transmitting material (111) and the first light-shielding material (141), to obtain the first composite rod body; and/or
the providing (210) a second composite material (182) comprises:
providing the second light-transmitting material (121) and the second light-shielding material (151), and disposing the second light-shielding material (151) on a periphery of the second light-transmitting material (121); and
squeezing the second light-transmitting material (121) and the second light-shielding material (151), to obtain the second composite rod body.

5. The method as claimed in claim 4, wherein the first light-shielding material (141) is a single first opaque rod material, the first light-shielding material (141) defines a second hollow structure, and the disposing the first light-shielding material (141) on a periphery of the first light-transmitting material (111), comprises: disposing the first light-transmitting material (111) in the second hollow structure; or
the first light-shielding material (141) comprises a plurality of first opaque fiber filaments, and the disposing the first light-shielding material (141) on a periphery of the first light-transmitting material (111), comprises: arranging the plurality of first opaque fiber filaments on the periphery of the first light-transmitting material (111).

6. The method as claimed in claim 1, wherein the first light-transmitting material (111) is a single first light-transmitting rod material, or the first light-transmitting material (111) comprises a plurality of first light-transmitting fiber filaments; and /or
the second light-transmitting material (121) is a single second light-transmitting rod material, or the second light-transmitting material (121) comprises a plurality of second light-transmitting fiber filaments.

7. The method as claimed in claim 1, wherein
the treating (240) the composite rod material (170) to obtain a cover plate (10), comprises:
pulling the composite rod material (170), to obtain a pulled composite rod material; and
treating the pulled composite rod material, to obtain the cover plate (10); or
the treating (240) the composite rod material (170) to obtain a cover plate (10), comprises:
annealing the composite rod material (170), to obtain an annealed composite rod material; and
treating the annealed composite rod material, to obtain the cover plate (10); or
the treating (240) the composite rod material (170) to obtain a cover plate (10), comprises:
pulling the composite rod material (170), to obtain the pulled composite rod material;
annealing the pulled composite rod material, to obtain an annealed composite rod material; and
treating the annealed composite rod material, to obtain the cover plate (10).

8. A cover plate (10), comprising:
a first window area (11) configured to allow light rays to pass through;
a second window area (12) configured to allow light rays to pass through, wherein the first window area (11) is spaced apart from the second window area (12);
a first light-shielding area (14) configured to shield light rays, wherein the first light-shielding area (14) surrounds the first window area (11);
a second light-shielding area (15) configured to shield light rays, wherein the second light-shielding area (15) surrounds the second window area (12); and
a peripheral area (16) surrounding the first light-shielding area (14) and the second light-shielding area (15);
**characterized in that** the cover plate (10) is formed by squeezing a first composite material (181), a second composite material (182), and a peripheral material (161); the first composite material (181) comprises a first light-transmitting material (111) and a first light-shielding material (141), and the first light-shielding material (141) surrounds the first light-transmitting material (111); the second composite material (182) comprises a second light-transmitting material (121) and a second light-shielding material (151), and the second light-shielding material (151) surrounds the second light-transmitting material (121); and
after squeezing, the first light-transmitting material (111) forms the first window area (11), the second light-transmitting material (121) forms the second window area (12), the first light-shielding material (141) forms the first light-shielding area (14), the second light-shielding material (151) forms the second light-shielding area (15), and the peripheral material (161) forms the peripheral area (16).

9. The cover plate (10) as claimed in claim 8, wherein a difference between a thermal expansion coefficient of a material of the first window area (11) and a thermal expansion coefficient of a material of the peripheral area (16) is defined as a first difference, a difference between the thermal expansion coefficient of the material of the first window area (11) and a thermal expansion coefficient of a material of the first light-shielding area (14) is defined as a second difference, and an absolute value of the first difference is less than or equal to an absolute value of the second difference; and/or
a difference between a thermal expansion coefficient of a material of the second window area (12) and the thermal expansion coefficient of the material of the peripheral area (16) is defined as a third difference, a difference between the thermal expansion coefficient of the material of the second window area (12) and a thermal expansion coefficient of a material of the second light-shielding area (15) is defined as a fourth difference, and an absolute value of the third difference is less than or equal to an absolute value of the fourth difference.

10. The cover plate (10) as claimed in claim 8, wherein a material of the first window area (11), a material of the second window area (12), and a material of the peripheral area (16) are the same material; or a material of the first light-shielding area (14), a material of the second light-shielding area (15), and a material of the peripheral area (16) are the same material.

11. The cover plate (10) as claimed in claim 8, wherein each of the first light-shielding material and the second light-shielding material is selected from at least one of opaque glass, opaque plastic, opaque ceramic, and metal; and the peripheral material is glass, plastic, ceramic, or the metal.

12. The cover plate (10) as claimed in claim 8, wherein a material of the first light-shielding area (14) comprises a first opaque rod material, or the material of the first light-shielding area (14) comprises a plurality of first opaque fiber filaments; and
a material of the second light-shielding area (15) comprises a second opaque rod material, or the material of the second light-shielding area (15) comprises a plurality of second opaque fiber filaments.

13. The cover plate (10) as claimed in claim8, wherein a material of the first window area (11) comprises a first light-transmitting rod material, or the material of the first window area (11) comprises a plurality of first light-transmitting fiber filaments; and
a material of the second window area (12) comprises a second light-transmitting rod material, or the material of the second window area (12) comprises a plurality of second light-transmitting fiber filaments.

14. The cover plate (10) as claimed in claim 8, wherein each of a material of the first window area (11), a material of the second window area (12), a material of the first light-shielding area (14), a material of the second light-shielding area (15), and a material of the peripheral area (16) is glass; or
wherein the material of the first light-shielding area (14) comprises a plurality of first opaque fiber filaments arranged on a periphery of the first window area (11), and the material of the second light-shielding area (15) comprises a plurality of second opaque fiber filaments arranged on a periphery of the second window area (12).

15. An electronic device (100), **characterized by** comprising:
a cover plate (10) as claimed in any one of claims 8 to 14, or formed by any one method of claims 1 to 7;
a light emitter (20) disposed on a side of the cover plate (10), wherein light rays emitted by the light emitter (20) enter the first window area (11) and are transmitted to another side of the cover plate (10) away from the light emitter (20) through the first window area (11); and
a light detector (30), wherein the light detector (30) and the light emitter (20) are disposed on the same side of the cover plate (10), and the light rays that enter the second window area (12) from the side of the cover plate (10) away from the light detector (30) pass through the second window area (12) and enter the light detector (30).

## Patentansprüche

1. Verfahren zur Herstellung einer Abdeckplatte (10), umfassend:
Bereitstellen (210) eines ersten Verbundmaterials (181), eines zweiten Verbundmaterials (182) und eines Peripheriematerials (161), wobei das erste Verbundmaterial (181) ein erstes lichtdurchlässiges Material (111) und ein erstes Lichtabschirmmaterial (141) umfasst und das erste Lichtabschirmmaterial (141) das erste lichtdurchlässige Material (111) umgibt; und das zweite Verbundmaterial (182) ein zweites lichtdurchlässiges Material (121) und ein zweites Lichtabschirmmaterial (151) umfasst und das zweite Lichtabschirmmaterial (151) das zweite lichtdurchlässige Material (121) umgibt;
Anordnen (220) des ersten Verbundmaterials (181) und des zweiten Verbundmaterials (182) in Abständen, und Anordnen des Peripheriematerials (161) an einem Umfang des ersten Verbundmaterials (181) und einem Umfang des zweiten Verbundmaterials (182);
**dadurch gekennzeichnet, dass** das Verfahren zur Herstellung der Abdeckplatte (10) ferner das Zusammenpressen (230) des ersten Verbundmaterials (181), des zweiten Verbundmaterials (182) und des Peripheriematerials (161) umfasst, um ein Verbundstabmaterial (170) zu erhalten; und
Behandeln (240) des Verbundstabmaterials (170), um eine Abdeckplatte (10) zu erhalten.

2. Verfahren nach Anspruch 1, wobei das Peripheriematerial (161) ein einzelnes Stabmaterial ist und das Peripheriematerial (161) eine Vielzahl von ersten Hohlstrukturen (102) definiert, die in Abständen angeordnet sind; das Anordnen des Peripheriematerials (161) an einem Umfang des ersten Verbundmaterials (181) und einem Umfang des zweiten Verbundmaterials (182) umfasst: Anordnen des ersten Verbundmaterials (181) und des zweiten Verbundmaterials (182) in verschiedenen ersten Hohlstrukturen (102) jeweils; oder
das Peripheriematerial (161) eine Vielzahl von Faserfäden umfasst; das Anordnen des Peripheriematerials (161) an einem Umfang des ersten Verbundmaterials (181) und einem Umfang des zweiten Verbundmaterials (182) umfasst: Anordnen der Vielzahl von Faserfäden an dem Umfang des ersten Verbundmaterials (181) und dem Umfang des zweiten Verbundmaterials (182).

3. Verfahren nach Anspruch 1, wobei das erste Verbundmaterial (181) einen ersten Verbundstabkörper umfasst, der durch Kombinieren des ersten lichtdurchlässigen Materials (111) und des ersten Lichtabschirmmaterials (141) gebildet ist; und/oder
das zweite Verbundmaterial (182) einen zweiten Verbundstabkörper umfasst, der durch Kombinieren des zweiten lichtdurchlässigen Materials (121) und des zweiten Lichtabschirmmaterials (151) gebildet ist.

4. Verfahren nach Anspruch 3, wobei
das Bereitstellen (210) eines ersten Verbundmaterials (181) umfasst:
Bereitstellen des ersten lichtdurchlässigen Materials (111) und des ersten Lichtabschirmmaterials (141), und Anordnen des ersten Lichtabschirmmaterials (141) an einem Umfang des ersten lichtdurchlässigen Materials (111); und
Zusammenpressen des ersten lichtdurchlässigen Materials (111) und des ersten Lichtabschirmmaterials (141), um den ersten Verbundstabkörper zu erhalten; und/oder
das Bereitstellen (210) eines zweiten Verbundmaterials (182) umfasst:
Bereitstellen des zweiten lichtdurchlässigen Materials (121) und des zweiten Lichtabschirmmaterials (151), und Anordnen des zweiten Lichtabschirmmaterials (151) an einem Umfang des zweiten lichtdurchlässigen Materials (121);
und
Zusammenpressen des zweiten lichtdurchlässigen Materials (121) und des zweiten Lichtabschirmmaterials (151), um den zweiten Verbundstabkörper zu erhalten.

5. Verfahren nach Anspruch 4, wobei das erste Lichtabschirmmaterial (141) ein einzelnes erstes opakes Stabmaterial ist, das erste Lichtabschirmmaterial (141) eine zweite Hohlstruktur definiert, und das Anordnen des ersten Lichtabschirmmaterials (141) an einem Umfang des ersten lichtdurchlässigen Materials (111) umfasst: Anordnen des ersten lichtdurchlässigen Materials (111) in der zweiten Hohlstruktur; oder
das erste Lichtabschirmmaterial (141) eine Vielzahl von ersten opaken Faserfäden umfasst, und das Anordnen des ersten Lichtabschirmmaterials (141) an einem Umfang des ersten lichtdurchlässigen Materials (111) umfasst: Anordnen der Vielzahl von ersten opaken Faserfäden an dem Umfang des ersten lichtdurchlässigen Materials (111).

6. Verfahren nach Anspruch 1, wobei das erste lichtdurchlässige Material (111) ein einzelnes erstes lichtdurchlässiges Stabmaterial ist, oder das erste lichtdurchlässige Material (111) eine Vielzahl von ersten lichtdurchlässigen Faserfäden umfasst; und /oder
das zweite lichtdurchlässige Material (121) ein einzelnes zweites lichtdurchlässiges Stabmaterial ist, oder das zweite lichtdurchlässige Material (121) eine Vielzahl von zweiten lichtdurchlässigen Faserfäden umfasst.

7. Verfahren nach Anspruch 1, wobei
das Behandeln (240) des Verbundstabmaterials (170), um eine Abdeckplatte (10) zu erhalten, umfasst:
Ziehen des Verbundstabmaterials (170), um ein gezogenes Verbundstabmaterial zu erhalten; und
Behandeln des gezogenen Verbundstabmaterials, um die Abdeckplatte (10) zu erhalten; oder
das Behandeln (240) des Verbundstabmaterials (170), um eine Abdeckplatte (10) zu erhalten, umfasst:
Tempern des Verbundstabmaterials (170), um ein getempertes Verbundstabmaterial zu erhalten; und
Behandeln des getemperten Verbundstabmaterials, um die Abdeckplatte (10) zu erhalten; oder
das Behandeln (240) des Verbundstabmaterials (170), um eine Abdeckplatte (10) zu erhalten, umfasst:
Ziehen des Verbundstabmaterials (170), um das gezogene Verbundstabmaterial zu erhalten;
Tempern des gezogenen Verbundstabmaterials, um ein getempertes Verbundstabmaterial zu erhalten; und
Behandeln des getemperten Verbundstabmaterials, um die Abdeckplatte (10) zu erhalten.

8. Abdeckplatte (10), umfassend:
einen ersten Fensterbereich (11), der konfiguriert ist, um den Durchgang von Lichtstrahlen zu ermöglichen;
einen zweiten Fensterbereich (12), der konfiguriert ist, um den Durchgang von Lichtstrahlen zu ermöglichen, wobei der erste Fensterbereich (11) von dem zweiten Fensterbereich (12) beabstandet ist;
einen ersten Lichtabschirmbereich (14), der konfiguriert ist, um Lichtstrahlen abzuschirmen, wobei der erste Lichtabschirmbereich (14) den ersten Fensterbereich (11) umgibt;
einen zweiten Lichtabschirmbereich (15), der konfiguriert ist, um Lichtstrahlen abzuschirmen, wobei der zweite Lichtabschirmbereich (15) den zweiten Fensterbereich (12) umgibt; und
einen Peripheriebereich (16), der den ersten Lichtabschirmbereich (14) und den zweiten Lichtabschirmbereich (15) umgibt;
**dadurch gekennzeichnet, dass** die Abdeckplatte (10) durch Zusammenpressen eines ersten Verbundmaterials (181), eines zweiten Verbundmaterials (182) und eines Peripheriematerials (161) gebildet ist; das erste Verbundmaterial (181) ein erstes lichtdurchlässiges Material (111) und ein erstes Lichtabschirmmaterial (141) umfasst, und das erste Lichtabschirmmaterial (141) das erste lichtdurchlässige Material (111) umgibt; das zweite Verbundmaterial (182) ein zweites lichtdurchlässiges Material (121) und ein zweites Lichtabschirmmaterial (151) umfasst, und das zweite Lichtabschirmmaterial (151) das zweite lichtdurchlässige Material (121) umgibt; und
nach dem Zusammenpressen das erste lichtdurchlässige Material (111) den ersten Fensterbereich (11) bildet, das zweite lichtdurchlässige Material (121) den zweiten Fensterbereich (12) bildet, das erste Lichtabschirmmaterial (141) den ersten Lichtabschirmbereich (14) bildet, das zweite Lichtabschirmmaterial (151) den zweiten Lichtabschirmbereich (15) bildet, und das Peripheriematerial (161) den Peripheriebereich (16) bildet.

9. Abdeckplatte (10) nach Anspruch 8, wobei eine Differenz zwischen einem Wärmeausdehnungskoeffizienten eines Materials des ersten Fensterbereichs (11) und einem Wärmeausdehnungskoeffizienten eines Materials des Peripheriebereichs (16) als eine erste Differenz definiert ist, eine Differenz zwischen dem Wärmeausdehnungskoeffizienten des Materials des ersten Fensterbereichs (11) und einem Wärmeausdehnungskoeffizienten eines Materials des ersten Lichtabschirmbereichs (14) als eine zweite Differenz definiert ist, und ein Absolutwert der ersten Differenz kleiner oder gleich einem Absolutwert der zweiten Differenz ist; und/oder
eine Differenz zwischen einem Wärmeausdehnungskoeffizienten eines Materials des zweiten Fensterbereichs (12) und dem Wärmeausdehnungskoeffizienten des Materials des Peripheriebereichs (16) als eine dritte Differenz definiert ist, eine Differenz zwischen dem Wärmeausdehnungskoeffizienten des Materials des zweiten Fensterbereichs (12) und einem Wärmeausdehnungskoeffizienten eines Materials des zweiten Lichtabschirmbereichs (15) als eine vierte Differenz definiert ist, und ein Absolutwert der dritten Differenz kleiner oder gleich einem Absolutwert der vierten Differenz ist.

10. Abdeckplatte (10) nach Anspruch 8, wobei ein Material des ersten Fensterbereichs (11), ein Material des zweiten Fensterbereichs (12) und ein Material des Peripheriebereichs (16) dasselbe Material sind; oder ein Material des ersten Lichtabschirmbereichs (14), ein Material des zweiten Lichtabschirmbereichs (15) und ein Material des Peripheriebereichs (16) dasselbe Material sind.

11. Abdeckplatte (10) nach Anspruch 8, wobei jedes des ersten Lichtabschirmmaterials und des zweiten Lichtabschirmmaterials aus mindestens einem von opakem Glas, opakem Kunststoff, opaker Keramik und Metall ausgewählt ist; und das Peripheriematerial Glas, Kunststoff, Keramik oder das Metall ist.

12. Abdeckplatte (10) nach Anspruch 8, wobei ein Material des ersten Lichtabschirmbereichs (14) ein erstes opakes Stabmaterial umfasst, oder das Material des ersten Lichtabschirmbereichs (14) eine Vielzahl von ersten opaken Faserfäden umfasst; und
ein Material des zweiten Lichtabschirmbereichs (15) ein zweites opakes Stabmaterial umfasst, oder das Material des zweiten Lichtabschirmbereichs (15) eine Vielzahl von zweiten opaken Faserfäden umfasst.

13. Abdeckplatte (10) nach Anspruch 8, wobei ein Material des ersten Fensterbereichs (11) ein erstes lichtdurchlässiges Stabmaterial umfasst, oder das Material des ersten Fensterbereichs (11) eine Vielzahl von ersten lichtdurchlässigen Faserfäden umfasst; und
ein Material des zweiten Fensterbereichs (12) ein zweites lichtdurchlässiges Stabmaterial umfasst, oder das Material des zweiten Fensterbereichs (12) eine Vielzahl von zweiten lichtdurchlässigen Faserfäden umfasst.

14. Abdeckplatte (10) nach Anspruch 8, wobei jedes eines Materials des ersten Fensterbereichs (11), eines Materials des zweiten Fensterbereichs (12), eines Materials des ersten Lichtabschirmbereichs (14), eines Materials des zweiten Lichtabschirmbereichs (15) und eines Materials des Peripheriebereichs (16) Glas ist; oder
wobei das Material des ersten Lichtabschirmbereichs (14) eine Vielzahl von ersten opaken Faserfäden umfasst, die an einem Umfang des ersten Fensterbereichs (11) angeordnet sind, und das Material des zweiten Lichtabschirmbereichs (15) eine Vielzahl von zweiten opaken Faserfäden umfasst, die an einem Umfang des zweiten Fensterbereichs (12) angeordnet sind.

15. Elektronisches Gerät (100), **dadurch gekennzeichnet, dass** es umfasst:
eine Abdeckplatte (10) nach einem der Ansprüche 8 bis 14, oder gebildet durch eines der Verfahren der Ansprüche 1 bis 7;
einen Lichtemitter (20), der an einer Seite der Abdeckplatte (10) angeordnet ist, wobei von dem Lichtemitter (20) emittierte Lichtstrahlen in den ersten Fensterbereich (11) eintreten und zu einer anderen Seite der Abdeckplatte (10), die von dem Lichtemitter (20) entfernt ist, durch den ersten Fensterbereich (11) übertragen werden; und
einen Lichtdetektor (30), wobei der Lichtdetektor (30) und der Lichtemitter (20) an derselben Seite der Abdeckplatte (10) angeordnet sind, und die Lichtstrahlen, die in den zweiten Fensterbereich (12) von der Seite der Abdeckplatte (10), die von dem Lichtdetektor (30) entfernt ist, eintreten, den zweiten Fensterbereich (12) durchlaufen und in den Lichtdetektor (30) eintreten.

## Revendications

1. Procédé de fabrication d'une plaque de couverture (10), comprenant :
la fourniture (210) d'un premier matériau composite (181), d'un deuxième matériau composite (182) et d'un matériau périphérique (161), dans lequel le premier matériau composite (181) comprend un premier matériau transmettant la lumière (111) et un premier matériau de blindage contre la lumière (141), et le premier matériau de blindage contre la lumière (141) entoure le premier matériau transmettant la lumière (111) ; et le deuxième matériau composite (182) comprend un deuxième matériau transmettant la lumière (121) et un deuxième matériau de blindage contre la lumière (151), et le deuxième matériau de blindage contre la lumière (151) entoure le deuxième matériau transmettant la lumière (121) ;
la disposition (220) du premier matériau composite (181) et du deuxième matériau composite (182) à des intervalles, et la disposition du matériau périphérique (161) sur une périphérie du premier matériau composite (181) et une périphérie du deuxième matériau composite (182) ;
**caractérisé en ce que** le procédé de fabrication de la plaque de couverture (10) comprend en outre la compression (230) du premier matériau composite (181), du deuxième matériau composite (182) et du matériau périphérique (161), pour obtenir un matériau composite en barreau (170) ; et
le traitement (240) du matériau composite en barreau (170) pour obtenir une plaque de couverture (10).

2. Procédé selon la revendication 1, dans lequel le matériau périphérique (161) est un matériau en barreau unique, et le matériau périphérique (161) définit une pluralité de premières structures creuses (102) disposées à des intervalles ; la disposition du matériau périphérique (161) sur une périphérie du premier matériau composite (181) et une périphérie du deuxième matériau composite (182) comprend : la disposition du premier matériau composite (181) et du deuxième matériau composite (182) dans différentes premières structures creuses (102) respectivement ; ou
le matériau périphérique (161) comprend une pluralité de filaments de fibres ; la disposition du matériau périphérique (161) sur une périphérie du premier matériau composite (181) et une périphérie du deuxième matériau composite (182) comprend : la disposition de la pluralité de filaments de fibres sur la périphérie du premier matériau composite (181) et la périphérie du deuxième matériau composite (182).

3. Procédé selon la revendication 1, dans lequel le premier matériau composite (181) comprend un premier corps de barreau composite formé en combinant le premier matériau transmettant la lumière (111) et le premier matériau de blindage contre la lumière (141) ; et/ou
le deuxième matériau composite (182) comprend un deuxième corps de barreau composite formé en combinant le deuxième matériau transmettant la lumière (121) et le deuxième matériau de blindage contre la lumière (151).

4. Procédé selon la revendication 3, dans lequel
la fourniture (210) d'un premier matériau composite (181) comprend :
la fourniture du premier matériau transmettant la lumière (111) et du premier matériau de blindage contre la lumière (141), et la disposition du premier matériau de blindage contre la lumière (141) sur une périphérie du premier matériau transmettant la lumière (111) ; et
la compression du premier matériau transmettant la lumière (111) et du premier matériau de blindage contre la lumière (141), pour obtenir le premier corps de barreau composite ; et/ou
la fourniture (210) d'un deuxième matériau composite (182) comprend :
la fourniture du deuxième matériau transmettant la lumière (121) et du deuxième matériau de blindage contre la lumière (151), et la disposition du deuxième matériau de blindage contre la lumière (151) sur une périphérie du deuxième matériau transmettant la lumière (121) ;
et
la compression du deuxième matériau transmettant la lumière (121) et du deuxième matériau de blindage contre la lumière (151), pour obtenir le deuxième corps de barreau composite.

5. Procédé selon la revendication 4, dans lequel le premier matériau de blindage contre la lumière (141) est un premier matériau opaque en barreau unique, le premier matériau de blindage contre la lumière (141) définit une deuxième structure creuse, et la disposition du premier matériau de blindage contre la lumière (141) sur une périphérie du premier matériau transmettant la lumière (111) comprend : la disposition du premier matériau transmettant la lumière (111) dans la deuxième structure creuse ; ou
le premier matériau de blindage contre la lumière (141) comprend une pluralité de premiers filaments de fibres opaques, et la disposition du premier matériau de blindage contre la lumière (141) sur une périphérie du premier matériau transmettant la lumière (111) comprend : la disposition de la pluralité de premiers filaments de fibres opaques sur la périphérie du premier matériau transmettant la lumière (111).

6. Procédé selon la revendication 1, dans lequel le premier matériau transmettant la lumière (111) est un premier matériau en barreau unique transmettant la lumière, ou le premier matériau transmettant la lumière (111) comprend une pluralité de premiers filaments de fibres transmettant la lumière ; et /ou
le deuxième matériau transmettant la lumière (121) est un deuxième matériau en barreau unique transmettant la lumière, ou le deuxième matériau transmettant la lumière (121) comprend une pluralité de deuxièmes filaments de fibres transmettant la lumière.

7. Procédé selon la revendication 1, dans lequel
le traitement (240) du matériau composite en barreau (170) pour obtenir une plaque de couverture (10) comprend :
l'étirage du matériau composite en barreau (170), pour obtenir un matériau composite en barreau étiré ; et
le traitement du matériau composite en barreau étiré, pour obtenir la plaque de couverture (10) ; ou
le traitement (240) du matériau composite en barreau (170) pour obtenir une plaque de couverture (10) comprend :
le recuit du matériau composite en barreau (170), pour obtenir un matériau composite en barreau recuit ; et
le traitement du matériau composite en barreau recuit, pour obtenir la plaque de couverture (10) ; ou
le traitement (240) du matériau composite en barreau (170) pour obtenir une plaque de couverture (10) comprend :
l'étirage du matériau composite en barreau (170), pour obtenir le matériau composite en barreau étiré ;
le recuit du matériau composite en barreau étiré, pour obtenir un matériau composite en barreau recuit ; et
le traitement du matériau composite en barreau recuit, pour obtenir la plaque de couverture (10).

8. Plaque de couverture (10), comprenant :
une première zone de fenêtre (11) configurée pour permettre le passage de rayons lumineux ;
une deuxième zone de fenêtre (12) configurée pour permettre le passage de rayons lumineux, dans laquelle la première zone de fenêtre (11) est espacée de la deuxième zone de fenêtre (12) ;
une première zone de blindage contre la lumière (14) configurée pour bloquer les rayons lumineux, dans laquelle la première zone de blindage contre la lumière (14) entoure la première zone de fenêtre (11) ;
une deuxième zone de blindage contre la lumière (15) configurée pour bloquer les rayons lumineux, dans laquelle la deuxième zone de blindage contre la lumière (15) entoure la deuxième zone de fenêtre (12) ; et
une zone périphérique (16) entourant la première zone de blindage contre la lumière (14) et la deuxième zone de blindage contre la lumière (15) ;
**caractérisée en ce que** la plaque de couverture (10) est formée par compression d'un premier matériau composite (181), d'un deuxième matériau composite (182) et d'un matériau périphérique (161) ; le premier matériau composite (181) comprend un premier matériau transmettant la lumière (111) et un premier matériau de blindage contre la lumière (141), et le premier matériau de blindage contre la lumière (141) entoure le premier matériau transmettant la lumière (111) ; le deuxième matériau composite (182) comprend un deuxième matériau transmettant la lumière (121) et un deuxième matériau de blindage contre la lumière (151), et le deuxième matériau de blindage contre la lumière (151) entoure le deuxième matériau transmettant la lumière (121) ; et
après compression, le premier matériau transmettant la lumière (111) forme la première zone de fenêtre (11), le deuxième matériau transmettant la lumière (121) forme la deuxième zone de fenêtre (12), le premier matériau de blindage contre la lumière (141) forme la première zone de blindage contre la lumière (14), le deuxième matériau de blindage contre la lumière (151) forme la deuxième zone de blindage contre la lumière (15), et le matériau périphérique (161) forme la zone périphérique (16).

9. Plaque de couverture (10) selon la revendication 8, dans laquelle une différence entre un coefficient de dilatation thermique d'un matériau de la première zone de fenêtre (11) et un coefficient de dilatation thermique d'un matériau de la zone périphérique (16) est définie comme une première différence, une différence entre le coefficient de dilatation thermique du matériau de la première zone de fenêtre (11) et un coefficient de dilatation thermique d'un matériau de la première zone de blindage contre la lumière (14) est définie comme une deuxième différence, et une valeur absolue de la première différence est inférieure ou égale à une valeur absolue de la deuxième différence ; et/ou
une différence entre un coefficient de dilatation thermique d'un matériau de la deuxième zone de fenêtre (12) et le coefficient de dilatation thermique du matériau de la zone périphérique (16) est définie comme une troisième différence, une différence entre le coefficient de dilatation thermique du matériau de la deuxième zone de fenêtre (12) et un coefficient de dilatation thermique d'un matériau de la deuxième zone de blindage contre la lumière (15) est définie comme une quatrième différence, et une valeur absolue de la troisième différence est inférieure ou égale à une valeur absolue de la quatrième différence.

10. Plaque de couverture (10) selon la revendication 8, dans laquelle un matériau de la première zone de fenêtre (11), un matériau de la deuxième zone de fenêtre (12) et un matériau de la zone périphérique (16) sont le même matériau ; ou un matériau de la première zone de blindage contre la lumière (14), un matériau de la deuxième zone de blindage contre la lumière (15) et un matériau de la zone périphérique (16) sont le même matériau.

11. Plaque de couverture (10) selon la revendication 8, dans laquelle chacun du premier matériau de blindage contre la lumière et du deuxième matériau de blindage contre la lumière est sélectionné parmi au moins l'un d'un verre opaque, d'un plastique opaque, d'une céramique opaque et d'un métal ; et le matériau périphérique est du verre, du plastique, de la céramique ou le métal.

12. Plaque de couverture (10) selon la revendication 8, dans laquelle un matériau de la première zone de blindage contre la lumière (14) comprend un premier matériau opaque en barreau, ou le matériau de la première zone de blindage contre la lumière (14) comprend une pluralité de premiers filaments de fibres opaques ; et
un matériau de la deuxième zone de blindage contre la lumière (15) comprend un deuxième matériau opaque en barreau, ou le matériau de la deuxième zone de blindage contre la lumière (15) comprend une pluralité de deuxièmes filaments de fibres opaques.

13. Plaque de couverture (10) selon la revendication 8, dans laquelle un matériau de la première zone de fenêtre (11) comprend un premier matériau en barreau transmettant la lumière, ou le matériau de la première zone de fenêtre (11) comprend une pluralité de premiers filaments de fibres transmettant la lumière ; et
un matériau de la deuxième zone de fenêtre (12) comprend un deuxième matériau en barreau transmettant la lumière, ou le matériau de la deuxième zone de fenêtre (12) comprend une pluralité de deuxièmes filaments de fibres transmettant la lumière.

14. Plaque de couverture (10) selon la revendication 8, dans laquelle chacun d'un matériau de la première zone de fenêtre (11), d'un matériau de la deuxième zone de fenêtre (12), d'un matériau de la première zone de blindage contre la lumière (14), d'un matériau de la deuxième zone de blindage contre la lumière (15) et d'un matériau de la zone périphérique (16) est du verre ; ou
dans laquelle le matériau de la première zone de blindage contre la lumière (14) comprend une pluralité de premiers filaments de fibres opaques disposés sur une périphérie de la première zone de fenêtre (11), et le matériau de la deuxième zone de blindage contre la lumière (15) comprend une pluralité de deuxièmes filaments de fibres opaques disposés sur une périphérie de la deuxième zone de fenêtre (12).

15. Dispositif électronique (100), **caractérisé en ce qu'**il comprend :
une plaque de couverture (10) selon l'une quelconque des revendications 8 à 14, ou formée par l'un quelconque des procédés des revendications 1 à 7 ;
un émetteur de lumière (20) disposé sur un côté de la plaque de couverture (10), dans lequel les rayons lumineux émis par l'émetteur de lumière (20) entrent dans la première zone de fenêtre (11) et sont transmis vers un autre côté de la plaque de couverture (10) éloigné de l'émetteur de lumière (20) à travers la première zone de fenêtre (11) ; et
un détecteur de lumière (30), dans lequel le détecteur de lumière (30) et l'émetteur de lumière (20) sont disposés sur le même côté de la plaque de couverture (10), et les rayons lumineux qui entrent dans la deuxième zone de fenêtre (12) depuis le côté de la plaque de couverture (10) éloigné du détecteur de lumière (30) traversent la deuxième zone de fenêtre (12) et entrent dans le détecteur de lumière (30).
